(19) Europäisches Patentamt
European Patent Office
Office européen des brevets

(11) Veröffentlichungsnummer: **0 128 483**
**B1**

(12) # EUROPÄISCHE PATENTSCHRIFT

(45) Veröffentlichungstag der Patentschrift: **12.09.90**

(51) Int. Cl.[5]: **A 61 K 47/00,** A 61 K 9/20

(21) Anmeldenummer: **84106272.2**

(22) Anmeldetag: **01.06.84**

(54) **Neue galenische Zubereitungsformen von oralen Antidiabetika und Verfahren zu ihrer Herstellung.**

(30) Priorität: **08.06.83 DE 3320582**

(43) Veröffentlichungstag der Anmeldung:
**19.12.84 Patentblatt 84/51**

(45) Bekanntmachung des Hinweises auf die Patenterteilung:
**12.09.90 Patentblatt 90/37**

(84) Benannte Vertragsstaaten:
**AT BE CH DE FR IT LI LU NL SE**

(56) Entgegenhaltungen:
**EP-A-0 068 191**
**EP-A-0 086 468**
**DE-A-2 355 743**
**DE-A-3 100 535**
**DE-A-3 225 188**

(73) Patentinhaber: **Dr. Karl Thomae GmbH**
**Postfach 1755**
**D-7950 Biberach (Riss) (DE)**

(72) Erfinder: **Schepky, Gottfried, Dr.**
**Ulrich-von-Hutten-Weg 2**
**D-7950 Biberach 1 (DE)**
Erfinder: **Brickl, Rolf, Dr. Dipl.-Chem.**
**Erlenweg 37**
**D-7951 Warthausen (DE)**
Erfinder: **Rupprecht, Eckhard, Dr. Dipl.-Biol.**
**Riedbachstrasse 15**
**D-7960 Aulendorf-Tannhausen (DE)**
Erfinder: **Greischel, Andreas, Dr.**
**Dunantstrasse 5**
**D-7950 Biberach 1 (DE)**

**EP 0 128 483 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Courier Press, Leamington Spa, England.

**Beschreibung**

Die Erfindung betrifft neue galenische Zubereitungsformen von oralen Antidiabetika und Verfahren zu ihrer Herstellung.

Die oralen Antidiabetika enthalten als Wirkstoffe Sulfonylharnstoffe, wie Gliquidon, oder substituierte Phenylcarbonsäuren. Als weitere Sulfonylharnstoffe kommen unter anderem bevorzugt in Betracht Glibenclamid, Glibornurid, Glisoxepid, Glipizid und Gliclazid. Bei Gliquidon handelt es sich um den 1-Cyclohexyl-3-[[p-[2-(3,4-dihydro-7-methoxy-4,4-dimethyl-1,3-dioxo-2-(1H)-isochinolyl)-ethyl]-phenyl]-sulfonyl]harnstoff, der eine blutzuckersenkende Wirkung ausübt. Als weitere antidiabetisch wirkende Substanzen kommen auber auch in Frage: 4-[2-(Aroylamino)ethyl]benzoesäuren der allgemeinen Formel

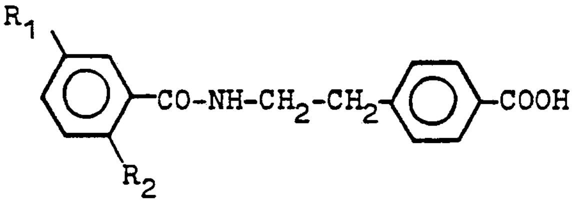

(I)

worin $R_1$ eine Halogenatom, vorzugsweise ein Chloratom und $R_2$ eine Alkoxygruppe mit 1 bis 3 Kohlenstoffatomen oder eine Piperidin-1-yl- oder die Octamethyleneiminogruppe bedeuten, desweiteren substituierte 4-(Aralkylaminocarbonylmethyl)-benzosäuren der allgemeinen Formel

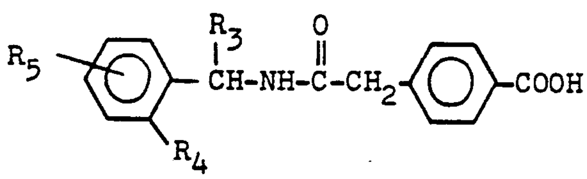

(II)

worin $R_3$ eine Alkylgruppe mit 1 bis 4 Kohlenstoffatomen, vorzugsweise die n-Propylgruppe, oder die Phenylgruppe, $R_4$ die Piperidin-1-yl-, Pyrrolidin-1-yl- oder Hexamethyleniminogruppe und $R_5$ eine Wasserstoff- oder Halogenatom, vorzugsweise ein Chlor- oder Fluoratom, oder die Methyl- oder Methoxygruppe darstellen. Es können aber auch Gemische dieser Wirkstoffe verwendet werden.

Mikronisiertes Gliquidon ist in einem sich im Handel befindenden Präparat, bestehend ansonsten aus Maisstärke, Milchzucker und Magnesiumstearat, enthalten. Dieses gliquidonhaltique Präparat hat sich bereits als sicheres Antidiabetikum bewährt mit dem großen Vorteil, daß es be eingeschränkter Nierenfunktion nicht konstraindiziert ist.

Im allgemeinen bestehen bei der oralen Applikation von in den Verdauungsflüssigkeiten schwerlöslichen Substanzen, und zu diesen zählen auch die oben genannten Substanzen, folgende Probleme: Der Wirkstoff kann in manchen Fällen nur unvollständig resorbiert werden und es können auch inter- und intraindividuell stark schwankende Blutspiegel des Wirkstoffes auftreten. Bei oralen Antidiabetika sind jedoch darüberhinaus der Wirkungseintritt und die Wirkungsdauer von besonderer Bedeutung, da die Wirkung auf die durch Nahrungsaufnahme hervorgerufenen Blutzuckerspiegel abgestimmt sein sollte. Diese ist mit den bisher zur Verfügung stehenden Zubereitungen von Antidiabetika, bei denen sich Substanzwirkung und physiologischer Insulinbedarf nach der Nahrungsaufnahme zeitlich nicht in zuverlässiger Weise aufeinander abstimmen lassen, nicht der Fall. Die Substanzwirkung setzt oft zu spät ein; oft wird die maximale Wirkung erst zu einem Zeitpunkt erreicht, zu welchem die Blutglukosewerte nach wenn der Nahrungsaufnahme auch ohne Medikation bereits abfallen. Danach hält die Substanzwerkung noch an, die Blutglukose bereits wieder den Ausgangswert erreicht (vgl. Berger, in Pelzer und Froesch, Diabetische Enteropathie, Hypoglykämien, Verlag Hans Hüber, Bern-Stuttgart-Wien 1974).

Es ist auch worden, die blutzuckersenkende Wirkung eines Sulfonylharnstoffes mit dem nahrungsbedingten Anstieg des Bluckzuckers zu synchronisieren, indem der Sulfonylharnstoff entsprechende Zweit vor der Mahlzeit eingenommen wurde. Dabei zeigte sich aber, daß eine Einnahme des Wirkstoffes 30 Minuten vor der Mahlzeit zu keiner befriedigenden Wirkungsverbesserung führte (vgl. Sartor et al., Eur. J. Clin. Pharmacolog. *21*, 403 bis 408 (1982)), u.a. wegen der oben genannten längeren Wirkungsdauer. Eine bestimmte Zeitdifferenz zwischen Einnahme de Medikamments und der Nahrungsaufnahme läßt sich zudem nur in einer Klinik zuverlässig kontrollieren.

Man versuchte, diese Probleme bei Substanzen, die in den Verdauungsflüssigkeiten schwer löslich sind, dadurch zu lösen, daß man bestrebt war, bei der Entwicklung der galenischen Zubereitungen das Lösungsverhalten (Dissolution Rate) des für sich allein schwerlöslichen Wirkstoffes zu optimieren. Dies gelang beispielsweise durch eine Vergrößerung der Oberfläche des Wirkstoffes. So wird eine Zubereitungsform beschrieben (DE—PS 2 348 334), worin der Wirkstoff (auch ein blutzuckersenkender Stoff) mit einer Teilchenoberfläche von 3 bis 10 $m^2/g$ in Gegenwart eines Netzmittels vorliegt.

Dieses Ziel sollte aber auch dadurch erreicht werden, daß der Wirkstoff in gelöster Form auf einen Träger mit möglichst großer Oberfläche aufgezogen und das Lösungsmittel entfernt wird (vgl. H.

Rupprecht, Acta Pharm. Technol. *26/1,* Seite 13 u.f. (1980)).

Es wurde darüberhinaus versucht, die Dissolution Rate durch den Zusatz von Salzbildnern zu verbassern (vgl. DE—OS 31 24 090.9). Zur Verbesserung der Löslichkeit und der Auflösungsgeschwindigkeit wurden aber auch feste Dispersionen hergestellt. Sie bestehen aus dem Wirkstoff und einem oder mehreren wasserlöslichen Trägern, gegebenenfalls noch in Kombination mit oberflächenaktiven Stoffen. Zur Herstellung dieser Dispersionen wird eine homogene Schmelze aus dem Wirkstoff oder gegebenenfalls dessen Salz und einem Träger hergestellt (vgl. DE—OS 23 55 743). Bei einem anderen Verfahren werden Wirkstoff und Träger in einem gemeinsamen Lösungsmittel gelöst, anschließend wird das Lösungsmittel entfernt. Als wasserlösliche Träger werden u.a. Polyvinylpyrrolidon bzw. Polyethylenglykole verwendet (vgl. H. R. Merkle, Acta Pharm. Technol *27/4,* S. 193 u.f. (1981) und W. L. Chiou, S. Riegelmann, J. Pharm. Sci. *60/9,* 1281 u.f. (1971)).

Wendet man die nachstehenden, in der Literatur beschriebenen Verfahrensweisen für die Herstellung von die eingangs genannten Verbindungen enthaltenden Zubereitungsformen an, so erzielt man kaum eine bessere Dissolution Rate des Wirkstoffes, z.B. des Gliquidons: die Salzbildung für sich allein bringt keine Erhöhung der Dissolution Rate (vgl. Tabelle 3, Beispiel 6), das Auftragen von Wirkstoff, z.B. von Gliquidon, auf einen Träger allein (vgl. Seite 8, Zeilen 12—24) erbringt ebenfalls nicht den gewünschten Erfolg. Bei entsprechenden Versuchen, die später noch eingehend geschildert werden, wurde die Dissolution Rate bestimmt und im Falle von Gliquidon als nicht großer gefunden als die Auflösungsgeschwindigkeiten, die an sich bekannte gliquidonhaltige Zubereitungsformen zeigen.

Es wurde nun überraschenderweise gefunden, daß sich die erfindungsgemäßen Zubereitungsformen mit einer sehr raschen und vollständigen Freigabe des Wirkstoffes dadurch herstellen lassen, daß

a) sauer, reagierende Wirkstoffe mit Hilfe basischer Hilfsstoffe,

b) amphoter reagierende Wirkstoffe mit Hilfe basischer oder saurer Hilfsstoffe

c) basisch reagierende Wirkstoffe mit Hilfe saurer Hilfsstoffe

in Gegenwart eines der mehrerer lösungsvermittelnder Stoffe, in einem Lösungsmittel gelöst werden. Als Lösungsvermittler dienen Polyvinylpyrrolidon und, gegebenenfalls, andere lösungsvermittelnde Stoffe. Die Lösung wird zur Trockne eingedamptf und, gegebenenfalls, zu gewünschten Zubereitungsformen weiterverarbeitet. Gegenstand der Erfindung sind aber auch die so gewonnen Zubereitungsformen selbst. In jedem Fall muß aber das molare Verhältnis von Wirkstoff zu basischen bzw. saurem Hilfsstoff so gewählt werden, daß ein Überschuß an basischen bzw. saurem Hilfsstoff vorliegt.

Wichtig ist, daß soviel an basischen bzw. saurem Hilfstoff dem Wirkstoff zugesetzt wird, daß in vivo eine rasche und vollständige Auflösung erfolgt. Dies ist erst bei einem molaren Verhältnis Wirkstoff zu basischen bzw. saurem Hilfsstoff kleiner als 1:1 möglich.

Um beispielsweise 2,5 Gewichtsteile Gliquidon in 50 Gewichtsteilen Wasser in Lösung zu bringen, sind 0,7 Gewichtsteile Ethylendiamin x $1H_2O$, 3,0 Gewichtsteile N-Methylglucamin oder 3,5 Gewichtsteile Diethanolamin notwendig.

Vergleicht man die molaren Verhältnisse, die unbedingt nötig sind für eine ransche und vollständige Auflösung des Wirkstoffes, so ergibt sich folgendes Bild:

Gliquidon (Mol.gew. 527,6: Ethylendiamin x $1H_2O$ (Mol.gew. 78,1) wie 1:1,89;

Gliquidon: N-Methylglucamin (Mol.gew. 195,21) wie 1:3,24;

Gliquidon: Diethanolamin (Mol.gew. 105,14) wie 1:7,03;

Gliquidon: L-Lysin (Mol.gew. 146,2) wie 1:4,33.

Dieser Befund läßt sich mit einer reinen Salzbildung des Gliquidons mit den jeweiligen basischen Hilfsstoffen nicht erklären; es scheint, daß die überschüssige Base einen zusätzlichen stabilisierenden Effekt ausübt. Dieselben Erscheinungen treffen auch für die übrigen Wirkstoffe zu. Dieser Effekt war auch für den Fachman nicht vorhersehbar.

Die Lösungen werden mit Polyvinylpyrrolidon als Lösungsvermittler hergestellt, dieser Stoff dient nach dem Eindampfen gleichzeitig auch als Träger. Eine direkte Einarbitung von Wirkstoff und basischem Hilfsstoff in eine Schmelze von Polyvinylpyrrolidon scheitert daran, daß dieser Träger sich schon von Erreichen des Schmelzpunktes zersetzt.

Als basische Hilfsstoffe eignen sich eine Reihe anorganischer oder organischer Basen, die mindestens in den angewandten Dosisbereichen physiologisch unbedenklich sind, beispielsweise Natronlage, Kalilauge, Ammoniak, tert.Natriumphosphat, Diethanolamin, Ethylendiamin, N-Methylglucamin oder L-Lysin. Das molare Verhältnis von Wirkstoff zu basischem Hilfsstoff bzw. Hilfsstoffgemischen beträgt vorzugsweise 1:1,1 bis 1:10, eine darüberhinhausgehender Überschuß an Base kann aber in manchen Fällen auch von Vorteil sein.

Als saure Hilfsstoffe kommen Schwefelsäure oder Phosphorsäure in Frage, es muß ein Überschuß de Säure vorliegen.

Um so hoch konzentrierte Lösungen, wie sie bei der Applikation eines erfindungsemäßen Präparates offensichtlich erzeugt werden, zu stabilisieren, ist der Zusatz von Polyvinylpyrrolidon als lösungsvermittelnder und/oder emulgierender Stoff notwendig. Als weitere solche Stoffe kommen zusätzlich u.a. Polyoxyethylen-polyoxypropylen-Polymerisate, Polyethylenglykol 4000 oder 6000, polyäthoxylierte Sorbitanmonooleate, Sorbit, Glycerin-Polyethylenglykoloxystearate und Polyoxyethylenfettalkoholether in Frage. Dabei spielen sowohl die Art des lösungsvermittelnden Stoffes als such die jeweiligen Mengenverhältnisse eine wichtige Rolle für die Dissolution Rate des Wirkstoffes. Das Verhaltnis

von Wirkstoff, z.B. Gliquidon zur Gesamtmenge lös ungsvermittelnder Stoffe beträgt vorzugsweise 1:1 bis 1:10.

Zur Herstellung der Lösung von Wirkstoff, basischen oder sauren Hilfsstoffen sowie lösungsvermittelnden und/oder emulgierenden Stoffen dienen in erster Linie Wasser oder andere polare Lösungsmittel, wie niedere Alkohole, z.B. Ethanol, Isopropanol, Ketone, wie Azeton oder Gemische dieser Stoff mit Wasser.

Durch die Verwendung der erfindungsgemäßen Lösungsmethode anstelle des aus der DE—OS 23 55 743 bekannten Schmelzerfahrens zur Einarbeitung des Wirkstoffes, läßt sich dos nichtschmelzbare, lösungsvermittelnde Polyvinylpyrrolidon molekulardispers zusammen mit den Wirkstoffen verarbeiten.

Die vorstehend geschilderte Lösung des Problems ist aus den folgenden Gründen überraschend:

Die in de Literatur beschriebenen nachstehend aufgeführten Verfahrensweisen für die Einarbeitung von in den Verdauungsflüssigkeiten schwer löslichen Substanzen bewirken, angewandt auf die Herstellung von die vorstehend genannten Wirksubstanzen enthaltenden Zubereitungsformen, keine deutliche Erhöhung der Dissolution Rate der betreffenden Wirkstoffe; sie vermögen auch die für die im Handel sich befindenden gliquidonhaltigen Zubereitungsformen gefundenen Dissolution Rate nicht zu verbessern. Im folgenden werden einige diesbezügliche Versuche geschildert. Die Dissolution Rate wurdens nach 5 bzw. 30 Minuten nach der USP XX Paddle Methode in 900 ml Mc Ilvaine-Puffer, pH-Wert 7,0, bei 37°C und 100 Umdrehungen pro Minute bestimmt. Je Bestimmung wurde eine 40,0 mg Wirkstoff entsprechende Menge an Zubereitungsform eingesetzt, jede Bestimmung wurde 2-mal wiederholt und der Mittelwert aus den jeweiligen Ergebnissen berechnet.

Zur Bestimmung der Dissolution Rate bei einer Oberflächenvergrößerung von Gliquidon wurden 30 Gewichtsteile des Wirkstoffes in 150 Gewichtsteilen Methylenchlorid gelöst und die Lösung auf 210 Gewichtsteile eines Tablettenträgers aufgezogen. Nach dem Trocknen wurde der beaufschlagte Tablettenträger zu Tabletten verpreßt und die Dissolution Rate des Gliquidons aus diesens Tabletten bestimmt; nach 5 minuten lösten sich 5%, nach 30 Minuten 7% Wirkstoffes. Bei mikronisiertem Gliquidon ohne Hilfsstoffe waren nach 5 und nach 30 Minuten 0% gelöst. Verpresst man das mikronisierte Gliquidon zu Tabletten (siehe Vergleichsform des Beispiels 1), so waren nach 5 Minuten 5,8% und nach 30 Minuten 7,2% Wirkstoff gelöst.

Auch die Salzbildung des Gliquidons ergab keine günstigeren Dissolution Rates. In einer wäßrigen Lösung von 1,9 Gewichtsteile Ethylendiamin. $1H_2O$ wurden 5,0 Gewichtsteile Gliquidon unter Erwärmen und Rühren aufgelöst; diese Lösung wurde im Rotationsverdampfer unter Vakuum getrocknet und das fest Produkt durch ein Sieb der Maschenweite 1,0 mm gesiebt. Auch dieses Produkt ergab nach 5 Minuten und nach 30 Minuten eine Menge von nur 4% an gelösten Wirkstoff.

Nichteinmal die Verwendung eine gliquidonhaltigen Dispersion zeigte bessere Dissolution Rates. Analog zur der in der DE—OS 23 55 743 beschriebenen Methode wurden 1,47 Gewichtsteile Gliquidon in einer Schmelze aus 79,1 Gewichtsteilen Polyglykol 4000 und 5,0 Gewichtsteilen Polyoxyethylen-40-stearat gelöst und anschließend darin 14,43 Gewichtsteile Kaliumbikarbonat dispergiet. Die erstarrte Schmelze wurde durch ein Sieb der Maschenweite 1,0 mm gerieben. Die Bestimmung de Auflösungsrate ergab nich 5 Minuten 10% und nach 30 Minuten 7% an Wirkstoff.

In einer weiteren Versuchreihe wurde geprüft, ob de Einsatz von Gliquidon-Salzen in das in der DE—OS 2 355 743 beschriebene Verfahren zu besseren Auflösungsraten führt. Verwendet wurde wiederum eine Schmelze zus 79,1 Gewichtsteilen Polyethylenglykol 4000 und 5,0 Gewichtsteilen Polyoxyethylen-40-stearat, in der eine gesättigte Lösung des betreffenden Gliquidon-Salzes hergestellt wurde. Anschließend wurden in dieser Lösung 14,43 Gew.T. Kaliumhydrogencarbonat dispergiert. Die erstarrte Schmelze wurde durch ein Sieb der Maschenweite 1,0 gerieben.

Tabelle:

| Gliquidon-Salz mit | In der Schmelze aus PEG 4000 und Polyoxyethylen-40-stearat waren maximal an Wirkstoff löslich (umgerechnet als Base): | Für 30 mg Gliquidon pro Dosis werden an fester Lösung benötigt: |
|---|---|---|
| Ethylendiamin | 0,65 % | 4,6 g |
| $NH_4OH$ | 2,40 % | 1,25 g |
| Meglumine | 0,54 % | 5,54 g |
| Piperidin | 2,15 % | 1,395 g |
| NaOH | 1,99 % | 1,51 g |

(PEG 400 = Polyethylenglykol 4000)

Aus diesen Ergebnissen ist unschwer zu ersehen, daß die für 30 mg Gliquidon benötigte Menge an Schmelze in keiner schluckbaren und zerfallenden Tablette unterzubringen ist. Somit scheidet dieses Verfahren nach DE—OS 2 355 743 auch für Gliquidon-Salze sowie für die ähnlich nachdosierten Salze der anderen eingangs erwähnten Wirkstoff aus.

Wie aus den vorstehend geschilderten Versuchen ersichtlich wird, läßt sich die Erzielung einer raschen und vollständigen Auflösung der Wirkstoffe bei Anwendung der bekannten, für solche Zwecke als geeignet beschriebenen Methoden nicht erreichen.

Bei der Entwicklung von Arzneiformen erfolgt die Optimierung mit Hilfe von in vitro Methoden. Die Freisetzung und Auflösung des Wirkstoffes wird hierbei mit Hilfe von Dissolution Tests ermittelt. Um der in vivo Situation vergleichbare Verhältnisse zu schaffen, werden diese Tests normalerweise im Sauren bei pH 1,2 durchgeführt. Wendet man diesen pH bei den erfindungsgemäßen Formen an, so erhält man keine meßbaren Freisetzungsraten. In vitro Dissolution Tests müssen deshalb bei pH 7 (oder darüber) durchgeführt werden. Dies dürfte darauf zurückzuführen sein, daß die Löslichkeit des Wirkstoffs bei pH-Werten unter 7 nicht mehr ausreicht. Es mußte deshalb erwartet werden, daß auch in vivo im sauren Bereich des Intestinaltraktes nur eine relativ geringe Freisetzung des Wirkstoffes erfolgen würde. Die rasche und vollständige Resorption des Wirkstoffs auch im oberen Bereich des Intestinaltraktes ist deshalb auch für den Fachmann überraschend. Weiterhin ist überraschend, daß trotz der Unterschiede zwischen in vivo Situation und der Bestimmung der Dissolution Rate in vitro eine weitgehende Übereinstimmung zwischen in vitro und in vivo besteht. Dies zeigt sich beim Vergleich der Dissolution Rates des Beispiels 2 in Tabelle 1 mit dem Kurvenverlauf in Abbildung 1 einerseits und des Kurvenverlaufs der Form gemäß Beispiel 2 mit den Kurvenverläufen der Formen der nichterfindungsgemäßen Beispiele 6 und 8.

Werden nun die eingangs erwähnten blutzuckersenkenden Wirkstoffe nach den vorstehend beschriebenen Verfahren formuliert, so erhält man Arzneimittel, bei denen die Wirkung des Wirkstoffes auf den physiologischen Bedarf des Patienten an diesem Medikamment abstimmt ist. Diese besonderen Arzneimittel gewährleisten eine rasche und vollständige Resorption des Wirkstoffes. Eine rasche Resorption verkürzt die Zeit, die zwischen der Einnahme des Medikaments und der Einnahme der Mahlzeit liegen muß, um die blutzuckersenkende Wirkung des Sulfonylharnstoffes mit dem nahrungsbedingten Anstieg des Blutzuckers weitgehend zu synchronisieren. Eine rasche und Vollständige Resorption verringert intra- und interindividuelle Streuungen des Blutglukose-Spiegels, sie minimiert die Abhängigkeit der Resorption vom Zustand des Magen-Darmtraktes bzw. von Art und Menge der aufgenommenen Nahrung und gewährleistet damit eine bedarfsgerechte Stoffwechselführung und damit verbunden eine bedarfsgerechte Insulinausschüttung. Die eingangs beschriebenen Nachteile der derzeitig bekannten Formen werden bei Anwendung des erfindungsgemäßen Verfahrens vermieden.

Im Humanversuch (siehe Abb. 1) konnte der schnelle Wirkungseintritt der erfindungsgemäßen Form (Beispiel 2) und die geringe Wirkung der nicht erfindungsgemäßen Beispiele 6 und 8 gezeigt werden. Außerdem zeigt sich, daß in vitro und in vivo Werte gut übereinstimmen.

Die vorstehend genannten Befunde zeigen, daß die medizinische Zielsetzung:

a) Vermeidung eines unphysiologischen Blutzuckeranstieges nach Nahrungsaufnahme,

b) Vermeidung eines massiven Blutzuckerabfalles einige Stunden nach der Nahrungsaufnahme

mit den erfindungsgemäßen Präparaten erreicht wird.

Im folgenden sollen noch die angewandten Bestimmungsmethoden dargelegt werden:

Bestimmung der Blutglukose:

Der Blutzucker wurde im venösen Vollblut bestimmt. 50 µl Blut wurden mit 500 µl 0,32 m Perchlorsäure vom Eiweiß befreit. Nach Zentrifugation erfolgte die Messung der Glukose im Überstand nach der Hexokinase-Methode am Substratautomaten.

Humanuntersuchungen:

Die Blutentnahmen erfolgten, über Dauerkatheter mit heparinisierten Einmalspritzen. Nach einer Vorperiode von 15 Minuten, bei der der Verlauf des Blutzuckerspiegels bzw. des Insulinspiegels ohne Medikamment bestimmt wurde, wurde die galenische Zubereitung in Form eines Granulates bzw. als Tabletten un der jeweils vermerkten Dosierung mit 70 ml Wasser verabreicht.

Im folgenden soll auf des Wesen der Erfindung noch näher eingegangen werden, indem die aus den Versuchen der Beispiele erzielten besprochen werden.

Tabelle 1 zeigt dan Zusammenhang zwischen der Menge an Polyvinylpyrrolidon und der Dissolution Rate auf.

Man erkannt aus der Tabelle 2, daß des Vorliegen eines alkalischen Hilfsstoffes nicht nur für das In-Lösung-Bringen von Gliquidon sondern auch für die Erzielung einer raschung Freigabe des Wirkstoffes unbedingt notwendig ist. Um eine gleich hohe Dissolution Rate allein durch den Einsatz des Lösungsvermittelnden Trägers Kollidon 25® und ohne den Einsatz eines alkalischen Hilfsstoffes zu erzielen, müßte man die Menge an Kollidon 25® bei diesem Beispiel um fast eine Zehnerpotenz erhöhen. Eine derartig hoher Anteil an Polyvinylpyrrolidon scheidet aber aus praktischen Gründen aus (die Zubereitungsformen wären nicht mehr als orale Formen handhabbar, aber auch Herstellungs- und Kostengründe sprechen gegen die Schaffung derartiger Formen).

Es folgen die diesen Betrachtungen zugrundeliegenden Beispiele mit genauen Zahlenangaben:
In den Beispielen ist:
Kollidon 25® = Poly-N-vinylpyrrolidon(-2).
PEG 4000 = Polyethylenglykol 4000.
Pluronic F 68® = Polyoxyethylenpolyoxypropylen-Polymeres.
Avicel® = mikrokristalline Zellulose.
Explotab® = Natriumcarboxymethylstärke.
Amberlite IRP 88(R) = Kaliumsalz von Polymeren aus Methacrylsäure und Divinylbenzol.

Beispiele 1 bis 3

Die Tabelle 1 enthält die Beispiele 1 bis 3 mit ihren Auflösungsraten.

Tabelle 1:

Die Beispiele 1 bis 3 zeigen gemeinsam folgende Zusammensetzungen:
5 mg Gliquidon, 1,9 mg Ethylendiamin x $H_2O$.

| Beispiel | Kollidon 25(R) mg | Dissolution Rate in Prozent des in Lösung gegangenen Wirkstoffes nach 5 | und 30 Minuten |
|---|---|---|---|
| 1 | 10 | 91 | 93 |
| 2 | 30 | 95 | 97 |
| 3 | 60 | 92 | 96 |

Die Herstellung der Zubereitungsformen der Beispiele erfolgte so:

Das basische Hilfsmittel wird in 100 Gewichtsteilen Wasser von 70°C unter Rühren gelöst. Der Wirkstoff wird zugegeben; man rührt, bis dieser vollständig gelöst ist. In diese Lösung wird Polyvinylpyrrolidon gelöst. Die Lösung wird im Vakuum unter Rühren zur Trockene eingedampft und das Produkt durch ein Sieb von 1 mm Maschenweite gerieben.

Ein Vergleich mit einem bekannten gliquidonhaltigen Präparat folgender Zusammensetzung:

| | |
|---|---|
| mikronisiertes Gliquidon | 30 Gewichtsteile |
| Maisstärke | 75 Gewichtsteile |
| Milchzucker | 132 Gewichtsteile |
| Magnesiumstearat | 3 Gewichtsteile |

zeigte folgende Auflösungsrate:
nach 5 Minuten: 5,8% Gliquidon
nach 30 Minuten: 7,2% Gliquidon

Beispiele 4 und 5

Die in nachfolgender Tabelle 2 angeführten Zubereitungsformen besitzen neben 5 mg Gliquidon wachsende Mengen an Polyvinylpyrrolidon (Kollidon 25®), aber keinen basischen Hilfsstoff und keine genügende Menge an Trägerstoff. Erst ab der 6-fachen Menge an Kollidon 25® wird eine brauchbare Dissolution Rate erzielt.

Die Herstellung der Zubereitungsformen dieser Beispiele erfolgte durch gemeinsames Lösen des Wirkstoffes und des lösungsvermittelnden Stoffes in Ethanol; die Lösung wurde hernach zur Trockne eingedampft und das Produkt durch ein Sieb der Maschenweite 1 mm gerieben.

Tabelle 2:

| Beispiel | Kollidon 25(R) in mg | Dissolution Rate in Prozent des in Lösung gegangenen Wirkstoffes nach 5 | und 30 Minuten |
|---|---|---|---|
| 4 | 10 | 24 | 44 |
| 5 | 60 | 90 | 93 |

Biespiele 6 bis 8

Die Zubereitungsform des Beispiels 7 wurde wie im Beispiel 1 beschrieben hergestellt. Die Herstellung der Zubereitungsformen der Beispiele 6 und 8 erfolgte in Analogie zu der in den Beispielen 4 und 5 beschriebenen Methode.

Die Tabelle 3 zeigt die Zusammensetzungen und die jeweils gemessenen Dissolution Rates. Wie sich aus der Tabelle 3 ergibt, führt das alleinige Vorhandensein eines basischen Hilfsstoffes zu keiner brauchbaren Dissolution Rate, auch das alleinige Vorhandensein eines lösungsvermittelnden Stoffes (es fehlt der basische Hilfsstoff und der Trägerstoff) führt zu keinen Produkt mit brauchbarer Dissolution Rate. Hieraus wird ersichtlich, daß die Kombination Gliquidon mit basischem Hilfsstoff und Lösungsvermittler in Gegenwart eines löslichen Trägerstoffes, die besten Resultate bezüglich der raschen und möglichst vollständigen Auflösung des Wirkstoffes erbringt.

Tabelle 3:

| Beispiel | Gliquidon mg | basischer Hilfsstoff mg | 30 mg Kollidon 25® | Dissolution Rate in Prozent des in Lösung gegangenen Wirkstoffes nach 5 | und 30 Minuten |
|---|---|---|---|---|---|
| 6 | 5 | 1.9 } Ethylendiamin x $H_2O$ | nein | 4 | 4 |
| 7 | 25 | 6,5 } Ethylendiamin x $H_2O$ | ja | 88 | 94 |
| 8 | 25 | - | ja | 12 | 24 |
| 9 | 30 | 36,0 L-Lysin | ja (Kollidon 25 + Pluronic F 68) | 91 | |

Beispiel 9

1 Tablette enthält:

30,0 mg Gliquidon

36,0 mg L-Lysin

20,0 mg Kollidon 25

24,0 mg Pluronic F 68

Die Verarbeitung erfolgte analog den Beispielen 1—3, jedoch unter Eindampfen der Lösung in einem Sprühtrockner.

Pro Tablette werden hinzugefügt:

105,0 mg Avicel

105,0 mg Explotab

---

320,0 mg

Aus dieser Mischung werden runde, bikonvexe Tabletten von 320 mg und 10 mm Durchmesser gepreßt und mit Hydroxypropylmethylcellulose geschmacksabdeckend überzogen.

Dissolution rate: nach 5 Minuten: 91%.

Beispiel 10

Filmtabletten mit 4-[(1-(2-Piperidinio-phenyl)-1-butyl)-aminocarbonylmethyl]-benzoesäure

Beispiel 10a

1 Tablette enthält:

30 mg Wirkstoff

134 mg Amberlite IRP 88

134 mg Avicel

2 mg Magnesiumstearat

---

300 mg

Die Tablettenbestandteile werden miteinander vermischt, zu runden, bikonvexn Tabletten von 300 mg und 10 mm Durchmesser verpreßt und mit Hydroxypropylmethylcellulose geschmacksabdeckend überzogen.

Dissolution rate: nach 5 Minuten: 25,6%.
nach 30 Minuten: 36,3%.

Beispiel 10b

Wirkstoffgranulat

30 mg Wirkstoff des Biespiel 10a

36 mg L-Lysin

30 mg Kollidon 25

24 mg Pluronic F 68

Verarbeitung analog den Beispielen 1 bis 3. Diesem Granulat werden hinzugefügt:

90 mg Avicel

90 mg Amberlite IRP 88

---

300 mg

Aus dieser Mischung werden runde, bikonvexe Tabletten von 300 mg und 10 mm Durchmesser gepreßt und mit Hydroxypropylmethylcellulose geschmacksabdeckend überzogen.

Dissolution rate: nach 5 Minuten: 48,7%.
nach 30 Minuten: 81,3%.

Beispiel 11

Filmtabletten mit 4-[N-(α-2-Phenyl-2-piperidino-benzyl)-aminocarbonylmethyl]-benzoesäure

Beispiel 11a

1 Tablette enthält:

30 mg Wirkstoff

134 mg Amberlite IRP 88

134 mg Avicel

2 mg Magnesiumstearat

300 mg

Die Tablettenbestandteile werden miteinander vermischt, zu runden, bikonvexen Tabletten von 300 mg und 10 mm Durchmesser verpreßt und mit Hydroxypropylmethylcellulose geschmacksabdeckend überzogen.
Dissolution rate: nach 5 Minuten: 15,8%.
nach 30 Minuten: 20,9%.

Beispiel 11b

Wirkstoffgranulat

30 mg Wirkstoff des Biespiels 11a

36 mg L-Lysin

30 mg Kollidon 25

24 mg Pluronic F 68

Verarbeitung analog den Beispielen 1 bis 3. Diesem Granulat werden hinzugefügt:

93 mg Avicel

93 mg Amberlite IRP 88

300 mg

Aus dieser Mischung werden runde, bikonvexe Tabletten von 300 mg und 10 mm Durchmesser gepreßt und mit Hydroxypropylmethylcellulose geschmacksabdeckend überzogen.

Dissolution rate: nach 5 Minuten: 58,4%.
nach 30 Minuten: 93,4%.

Beispiel 12

Filmtabletten mit 4-[2-(5-Chlor-2-octamethylenimino-benzoylamino)ethyl]-benzoesäure

Beispiel 12a

1 Tablette enthält:

30 mg Wirkstoff

134 mg Amberlite IRP 88

134 mg Avicel

2 mg Magnesiumstearat

300 mg

Die Tablettenbestandteile werden miteinander vermischt, zu runden, bikonvexen Tabletten von 300 mg und 10 mm Durchmesser verpreßt und mit Hydroxypropylmethylcellulose geschmacksabdeckend überzogen.

Dissolution rate: nach 5 Minuten: 18,4%.
nach 30 Minuten: 27,2%.

Beispiel 12b

Wirkstoffgranulat

30 mg Wirkstoff des Biespiel 12a

36 mg L-Lysin

30 mg Kollidon 25

24 mg Pluronic F 68

Verarbeitung analog den Beispielen 1 bis 3. Diesem Granulat werden hinzugefügt:

90 mg Avicel

90 mg Amberlite IRP 88

300 mg

Aus dieser Mischung werden runde, bikonvexe Tabletten von 300 mg und 10 mm Durchmesser gepreßt und mit Hydroxypropylmethylcellulose geschmacksabdeckend überzogen.

Dissolution rate: nach 5 Minuten: 96,2%.
nach 30 Minuten: 99,9%.

Es folgt noch ein Beispiel für die Herstellung pharmazeutischer Zubereitungsformen:

Beispiel 13

Kapseln

Eine 10 mg Gliquidon entsprechende Menge von Granulat aus Beispiel Nr. 1 wird nach Zumischen einer entsprechenden Menge an Maisstärke und Magnesiumstearat in Hartgelatine-Kapseln der Größe 5 abgefüllt.

**Patentansprüche für die Vertragsstaaten: DE BE FR IT LU NL SE CH**

1. Verfahren zur Herstellung von oral antidiabetisch wirkenden Substanzen enthaltenden Zubereitungsformen, dadurch gekennzeichnet, daß sauer oder amphoter reagierende Wirkstoffe mit Hilfe basischer Hilfsstoffe in Gegenwart von Polyvinylpyrrolidon und, gegebenenfalls, anderen lösungsvermittelnden Stoffen in einem Lösungsmittel gelöst werden, wobei das molare Verhältnis von Wirkstoff zu basischem Hilfsstoff kleiner als 1:1 sein muß die Lösung zur Trockne eingedampft und, gegebenenfalls, zu gewünschten Zubereitungsförmen mit weiteren Hilfsstoffen weiterverarbeitet wird.

2. Verfahren nach Anspruch 1, dadurch gekennzeichnet, daß oral antidiabetisch wirkende Sulfonylharnstoffe verwendet werden.

3. Verfahren nach Anspruch 1, dadurch gekennzeichnet, daß als oral antidiabetisch wirkende Substanzen

a) 4-[2-(Aroylamino)ethyl]benzoesäuren der allgemeinen Formel

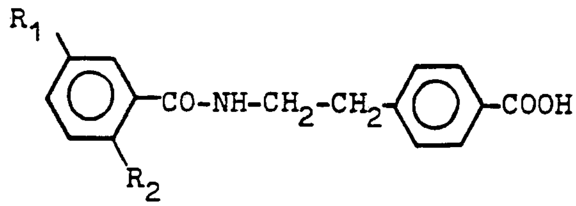

(I)

worin $R_1$ eine Halogenatom, vorzugsweise ein Chloratom und $R_2$ eine Alkoxygruppe mit 1 bis 3 Kohlenstoffatomen, oder eine Piperidin-1-yl- oder die Octamethyleniminogruppe bedeuten, oder

b) substituierte 4-(Aralkylaminocarbonylmethyl)-benzoesäuren der allgemeinen Formel

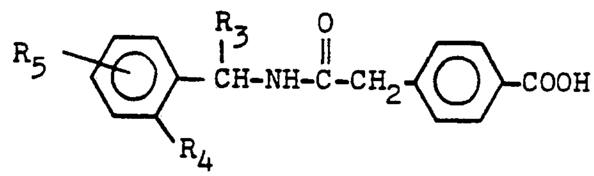

(II)

worin $R_3$ eine Alkylgruppe mit 1 bis 4 Kohlenstoffatomen, vorzugsweise die n-Propylgruppe, oder die Phenylgruppe, $R_4$ die Piperidin-1-yl-, Pyrrolidin-1-yl- oder Hexamethyleniminogruppe und $R_5$ eine Wasserstoff- oder Halogenatom, oder die Methyl- oder Methoxygruppe darstellen, oder Gemische dieser Stoffe verwendet werden.

4. Verfahren zur Herstellung von oral antidiabetisch wirkenden Zubereitungsformen nach Anspruch 1 enthaltend Gliquidon und/oder 4-[2-(5-Chlor-2-octamethyleniminobenzoylamino)ethyl]benzoesäure und/ oder 4-[N-(α-Phenyl-2-piperidino-benzyl)-aminocarbonylmethyl]-benzoesäure und/oder 4-[[1-(2-Piperidino-phenyl)-1-butyl]-aminocarbonylmethyl]-benzoesäure und einen löslichen Trägerstoff, dadurch gekennzeichnet, daß under Zusatz eines oder mehrerer basischer Hilfsstoffe der oder die Wirkstoffe in einem Lösungsmittel gelöst werden, wobei das molare Verhältnis von Wirkstoff zu basischem Hilfsstoff kleiner als 1:1 sein muß, darin Polyvinylpyrrolidon und, gegebenenfalls, andere lösungsvermittelnde Stoffe gelöst werden und diese Lösung zur Trockne eingedampft und, gebenenfalls, zu gewünschten Zubereitungsformen mit weiteren Hilfsttoffen weiterverarbeitet wird.

5. Verfahren nach den Ansprüchen 1 bis 4, dadurch gekennzeichnet, daß ein molares Verhältnis von Wirkstoff oder einem Gemisch der Wirkstoffe zu basischem Hilfsstoff von 1:1,1 bis 1:10 eingehalten wird.

6. Verfahren nach den Ansprüchen 1 bis 5, dadurch gekennzeichnet, daß der Wirkstoff oder ein Gemisch der Wirkstoffe und die Gesamtmenge lösungsvermittelnder Stoffe im Verhältnis von 1:1 bis 1:10 Gewichtsteilen eingesetzt werden.

7. Verfahren gemäß den Ansprüchen 1 bis 6, dadurch gekennzeichnet, daß als weitere lösungsvermittelnde Stoffe Poloxyethylen-polyoxypropylen-Polymerisate, Polyethylenglykole, polyethoxyliertes Sorbitanmonooleat, Sorbit, Glycerin-Polyethylenglykoloxystearate, Polyoxyethylenfettalkoholether oder Gemische dieser Stoffe, als basische Hilfsstoffe Natronlauge, Kalilauge, tert.-Natriumphosphat, Ammoniak, Diethanolamin, L-Lysin, Ethylendiamin oder N-Methylglucamin verwendet werden.

8. Oral antidiabetisch wirkende Substanzen enthaltende Zubereitungsformen, dadurch gekennzeichnet, daß sauer oder amphoter reagierende Wirkstoffe zusammen mit basisch reagierenden Hilfsstoffen wobei das molare Verhältnis von Wirkstoff zu basischem Hilfsstoff kleiner als 1:1 sein muß, an Polyvinylpyrrolidon gebunden vorliegt, gegebenenfalls noch in Anwesenheit weiterer lösungsvermittelnder, emulgierender oder sonstiger Hilfsstoffe, und das getrocknete Gut, gegebenenfalls zusammen mit weiteren Hilfsstoffen, zu entsprechenden Arzneiformen verarbeitet ist.

9. Antidiabetisch wirkende Zubereitungsformen nach Anspruch 8, enthaltend als weitere lösungsvermittelnde und/oder emulgierende Stoffe Poloxyethylen-polyoxypropylen-Polymerisate, Polyethylenglykole, polyethoxylierte Sorbitanmonooleat, Sorbit, Polyoxyethylenfettalkoholether, Glycerin-Polyethylenglykoloxystearate oder Gemische dieser Stoffe, als basische Hilfsstoffe Natronlauge, Diethanolamin, Ethylendiamin, L-Lysin oder N-Methylglucamin im Verhältnis von Wirkstoff zu basischem Hilfsstoff von 1:1,1 bis 1:10 Gewichtsteilen.

**Patentansprüche für den Vertragsstaat: AT**

1. Verfahren zur Herstellung von oral antidiabetisch wirkenden Substanzen enthaltenden Zubereitungsformen, dadurch gekennzeichnet, daß sauer oder amphoter reagierende Wirkstoffe mit Hilfe basischer Hilfsstoffe in Gegenwart von Polyvinylpyrrolidon und, gegebenenfalls, anderen lösungsvermittelnden Stoffen in einem Lösungsmittel gelöst werden, wobei das molare Verhältnis von Wirkstoff zu basischem Hilfsstoff kleiner als 1:1 sein muß, die Lösung zur Trockne eingedampft und,

gegebenenfalls, zu gewünschten Zubereitungsförmen mit weiteren Hilfsstoffen weiterverarbeitet wird.

2. Verfahren nach Anspruch 1, dadurch gekennzeichnet, daß oral antidiabetisch wirkende Sulfonylharnstoffe verwendet werden.

3. Verfahren nach Anspruch 1, dadurch gekennzeichnet, daß als oral antidiabetisch wirkende Substanzen

a) 4-[2-(Aroylamino)ethyl]benzoesäuren der allgemeinen Formel

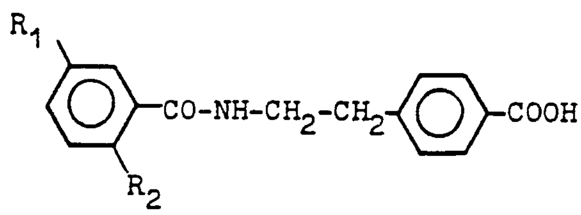

(I)

worin $R_1$ eine Halogenatom, vorzugsweise ein Chloratom und $R_2$ eine Alkoxygruppe mit 1 bis 3 Kohlenstoffatomen, oder eine Piperidin-1-yl- oder die Octamethyleniminogruppe bedeuten oder

b) substituierte 4-(Aralkylaminocarbonylmethyl)-benzoesäuren der allgemeinen Formel

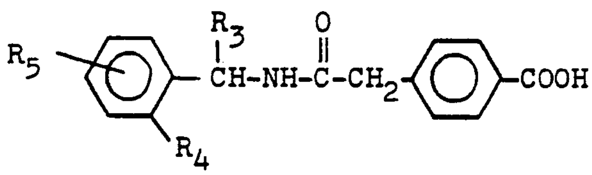

(II)

worin $R_3$ eine Alkylgruppe mit 1 bis 4 Kohlenstoffatomen, vorzugsweise die n-Propylgruppe, oder die Phenylgruppe, $R_4$ die Piperidin-1-yl-, Pyrrolidin-1-yl- oder Hexamethyleniminogruppe und $R_5$ eine Wasserstoff- oder Halogenatom oder die Methyl- oder Methoxygruppe darstellen, oder Gemische dieser Stoffe verwendet werden.

4. Verfahren zur Herstellung von oral antidiabetisch wirkenden Zubereitungsformen nach Anspruch 1 enthaltend Gliquidon und/oder 4-[2-(5-Chlor-2-octamethyleniminobenzoylamino)ethyl]benzoesäure und/oder 4-[N-(α-Phenyl-2-piperidino-benzyl)-aminocarbonylmethyl]-benzoesäure und/oder 4-[[1-(2-Piperidino-phenyl)-1-butyl]-aminocarbonylmethyl]-benzoesäure und einen löslichen Trägerstoff, dadurch gekennzeichnet, daß under Zusatz eines oder mehrerer basischer Hilfsstoffe der oder die Wirkstoffe in einem Lösungsmittel gelöst werden, wobei das molare Verhältnis von Wirkstoff zu basischem Hilfsstoff kleiner als 1:1 sein muß, darin Polyvinylpyrrolidon und, gegebenenfalls, andere lösungsvermittelnde Stoffe gelöst werden und diese Lösung zur Trockne eingedampft und, gebenenfalls, zu gewünschten Zubereitungsformen mit weiteren Hilfsttoffen weiterverarbeitet wird.

5. Verfahren nach den Ansprüchen 1 bis 4, dadurch gekennzeichnet, daß ein molares Verhältnis von Wirkstoff oder einem Gemisch der Wirkstoffe zu basischem Hilfsstoff von 1:1,1 bis 1:10 eingehalten wird.

6. Verfahren nach den Ansprüchen 1 bis 5, dadurch gekennzeichnet, daß der Wirkstoff oder ein Gemisch der Wirkstoffe und die Gesamtmenge lösungsvermittelnder Stoffe im Verhältnis von 1:1 bis 1:10 Gewichtsteilen eingesetzt werden.

7. Verfahren gemäß den Ansprüchen 1 bis 6, dadurch gekennzeichnet, daß als weitere lösungsvermittelnde Stoffe Poloxyethylen-polyoxypropylen-Polymerisate, Polyethylenglykole, polyethoxyliertes Sorbitanmonooleat, Sorbit, Glycerin-Polyethylenglykoloxystearate, Polyoxyethylenfettalkoholether oder Gemische dieser Stoffe, als basische Hilfsstoffe Natronlauge, Kalilauge, tert.-Natriumphosphat, Ammoniak, Diethanolamin, L-Lysin, Ethylendiamin oder N-Methylglucamin verwendet werden.

**Revendications pour les Etats contractants: DE BE FR IT LU NL SE CH**

1. Procédé pour la fabrication de formes de préparations contenant des substances à activité antidiabétique par voie orale, caractérisé en ce que des matières actives réagissant de façon acide ou amphotère sont dissoutes dans un solvant à l'aide d'adjuvants basiques en présence de polyvinylpyrrolidone et, éventuellement, d'autres agents de solubilisation, le rapport molaire de la matière active à l'adjuvant basique devant être inférieur à 1:1, la solution est évaporée jusqu'à siccité et, éventuellement, transformée encore en formes de préparations désirées avec des adjuvants supplémentaires.

2. Procédé selon la revendication 1, caractérisé en ce que l'on utilise des sulfonylurées à activité antidiabétique par voie orale.

3. Procédé selon la revendication 1, caractérisé en ce que l'on utilise, en tant que substances à activité antidiabétique par voie orale

a) des acides 4-[2-(aroylamino)éthyl]-benzoïques de formule générale

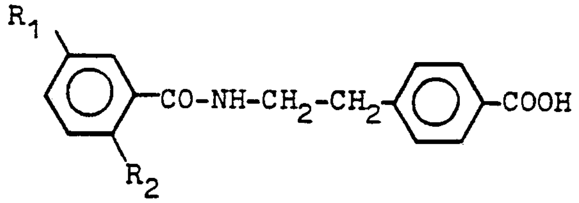

(I)

dans laquelle $R_1$ représente un atome d'halogène, de préférence un atome de chlorure et $R_2$ représente un groupe alcoxy avec 1 à 3 atomes de carbone, ou un groupe pipéridine-1-yle ou le groupe octaméthylènimino ou

b) des acides 4-(aralcoylaminocarbonylméthyl)-benzoïques substitués de formule générale

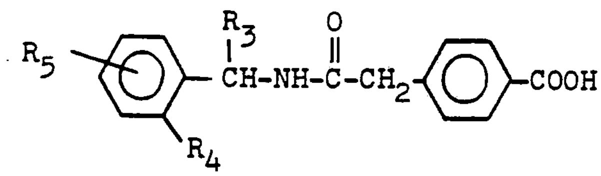

(II)

dans laquelle $R_3$ représente un groupe alcoyle avec 1 à 4 atomes de carbone, de préférence le groupe n-propyle, ou le groupe phényle, $R_4$ représente le groupe pipéridine-1-yle, pyrrolidone-1-yle ou hexaméthylènimino et $R_5$ représente un atome d'hydrogène ou d'halogène ou le groupe méthyle ou méthoxy, ou des mélanges de ces matières.

4. Procédé pour la fabrication de formes de préparations à activité antidiabétique par voie orale selon la revendication 1, contenant des la gliquidone et/ou de l'acide 4-[2-(5-chloro-2-octaméthylèniminobenzoyl-amino)éthyl]benzoïque et/ou de l'acide 4-[N-(α-phényl-2-pipéridino-benzyl)-aminocarbonylméthyl)-benzoïque et/ou de l'acide 4-[[1-(2-pipéridino-phényl)-1-butyl]-aminocarbonylméthyl]-benzoïque et un excipient soluble, caractérisé en ce qu'en présence d'un ou de plusieurs adjuvants basiques la ou les matières actives sont dissoutes dans un solvant, le rapport molaire de la matière active à l'adjuvant basique devant être inférieure à 1:1, on y dissout de la polyvinylpyrrolidone et, éventuellement, d'autres agents de solubilisation et cette solution est évaporée jusqu'à siccité et, éventuellement, transformée encore en des formes de préparation désirées à l'aide d'adjuvants supplémentaires.

5. Procédé selon les revendications 1 à 4, caractérisé en ce qu'on maintient un rapport molaire de la matière active ou d'un mélange des matières actives à l'adjuvant basique de 1:1,1 à 1:10.

6. Procédé selon les revendications 1 à 5 caractérisé en ce que la matière active ou un mélange des matières actives et la quantité total des agents de solubilisation sont mis en oeuvre dans le rapport de 1:1 à 1:10 parties en poids.

7. Procédé selon les revendications 1 à 6, caractérisé en ce qu'on utilise, en tant qu'agents de solubilisation supplémentaires, des polymères de polyoxyéthylène-polyoxypropylène, des polyéthylèneglycols, du monooléate de sorbitane polyéthoxylé, du sorbitol, du glycérol-polyéthylèneglycoloxystéarate, des éthers de polyoxyéthylène-alcools gras, ou des mélanges de ces matières, en tant qu'adjuvants basiques de la lessive de soude, de la lessive de potasse, du phosphate de tert.sodium, de l'amoniaque, de la diéthanolamine, de la L-lysine, de l'éthylènediamine ou de la N-méthylglucamine.

8. Formes de préparations contenant des substances à activité antidiabétique par voi orale, caractérisées en ce que des matières actives à réaction acide ou amphotère ensemble avec des adjuvants à réaction basique, le rapport molaire de la matière active à l'adjuvant basique devant être inférieure à 1:1, se présentent liées à de la polvyinylpyrrolidone, éventuellement encore en présence d'autres agents de solubilisation, adjuvants émulsifiants ou autres, et en ce que le produit séché, éventuellement ensemble avec d'autres adjuvants, est transformé en formes médicamenteuses correspondantes.

9. Formes de préparations à activité antidiabétique selon la revendication 8 contenant en tant qu'autres agents de solubilisation et/ou émulsifiants, des polymères de polyoxyéthylène-polyoxypropylène, des polyéthylèneglycols, du monooléate de sorbitane polyéthoxylé, du sorbitol, des éthers de polyoxyéthylène-alcools gras, du glycérol-polyéthylèneglycoloxystéarate ou des mélanges de ces matières, en tant qu'adjuvants basiques, de la lessive de soude, de la diéthanolamine, de l'éthylènediamine, la L-lysine, ou de N-méthylglucamine dans le rapport de la matière active à l'adjuvant basique 1:1,1 à 1:10 parties en poids.

**Revendications pour l'Etat contractant: AT**

1. Procédé pour la fabrication de formes de préparations contenant des substances à activité

antidiabétique par voie orale, caractérisé en ce que des matières actives réagissant de façon acide ou amphotère sont dissoutes dans un solvant à l'aide d'adjuvants basiques en présence de polyvinylpyrrolidone et, éventuellement, d'autres agents de solubilisation, le rapport molaire de la matière active à l'adjuvant basique devant être inférieur à 1:1, la solution est évaporée jusqu'à siccité et, éventuellement, transformée encore en formes de préparations désirées avec des adjuvants supplémentaires.

2. Procédé selon la revendication 1, caractérisé en ce que l'on utilise des sulfonylurées à activité antidiabétique par voie orale.

3. Procédé selon la revendication 1, caractérisé en ce que l'on utilise, en tant que substances à activité antidiabétique par voie orale

a) des acides 4-[2-(aroylamino)éthyl]-benzoïques de formule générale

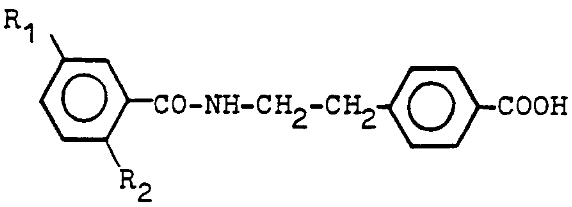
(I)

dans laquelle $R_1$ représente un atome d'halogène, de préférence un atome de chlorure et $R_2$ représente un groupe alcoxy avec 1 à 3 atomes de carbone, ou un groupe pipéridine-1-yle ou le groupe octaméthylènimino ou

b) des acides 4-(aralcoylaminocarbonylméthyl)-benzoïques substitués de formule générale

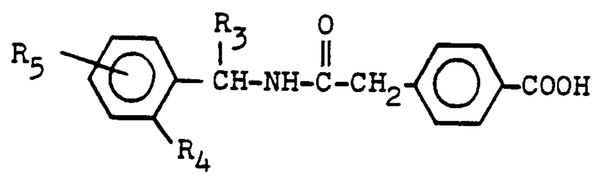
(II)

dans laquelle $R_3$ représente un groupe alcoyle avec 1 à 4 atomes de carbone, de préférence le groupe n-propyle, ou le groupe phényle, $R_4$ représente le groupe pipéridine-1-yle, pyrrolidine-1-yle ou hexaméthylènimino et $R_5$ représente un atome d'hydrogène ou d'halogène ou le groupe méthyle ou méthoxy, ou des mélanges de ces matières.

4. Procédé pour la fabrication de formes de préparations à activité antidiabétique par voie orale selon la revendication 1, contenant de la gliquidone et/ou de l'acide 4-[2-(5-chloro-2-octaméthylènimino-benzoylamino)éthyl]benzoïque et/ou de l'acide 4-[N-(α-phényle-2-pipéridino-benzyl)-aminocarbonyl-méthyl)-benzoïque et/ou de l'acide 4-[[1-(2-pipéridino-phényl)-1-butyl]-aminocarbonylméthyl]-benzoïque et un excipient soluble, caractérisé en ce qu'en présence d'un ou de plusieurs adjuvants basiques la ou les matières actives sont dissoutes dans un solvant, le rapport molaire de la matière active à l'adjuvant basique devant être inférieure à 1:1, on y dissout de la polyvinylpyrrolidone et, éventuellement, d'autres agents de solubilisation et cette solution est évaporée jusqu'à siccité et, éventuellement, transformée encore en des formes de préparation désirées à l'aide d'adjuvants supplémentaires.

5. Procédé selon les revendications 1 à 4, caractérisé en ce qu'on maintient un rapport molaire de la matière active ou d'un mélange des matières actives a l'adjuvant basique de 1:1,1 à 1:10.

6. Procédé selon les revendications 1 à 5, caractérisé en ce que la matière active ou un mélange des matières actives et la quantité total des agents de solubilisation sont mis en oeuvre dans le rapport de 1:1 à 1:10 parties en poids.

7. Procédé selon les revendications 1 à 6, caractérisé en ce que on utilise, en tant qu'agents de solubilisation supplémentaires, des polymères de polyoxyéthylène-polyoxypropylène, des polyéthylèneglycols, du monooléate de sorbitane polyéthoxylé, du sorbitol, du glycérol-polyéthylèneglycoloxystéarate, des éthers de polyoxyéthylène-alcools gras, ou des mélanges de ces matières, en tant qu'adjuvants basiques de la lessive de soude, de la lessive de potasse, du phosphate de tert.sodium, de l'amoniaque, de la diéthanolamine, de la L-lysine, de l'éthylènediamine ou de la N-méthylglucamine.

**Claims for the Contracting States: DE BE FR IT LU NL SE CH**

1. Process for producing oral preparation forms containing antidiabetically active substances, characterised in that acidically or amphoterically reacting active substances are dissolved with the aid of basic excipients in the presence of polyvinylpyrrolidone and optionally other solubilising substances in a solvent, whilst the molar ratio of active substance to basic excipient must be less than 1:1, the solution is evaporated to dryness and optionally further processed with other excipients to produce the desired preparation forms.

2. Process as claimed in claim 1, characterised in that oral antidiabetically active sulphonylureas are used.

3. Process as claimed in claim 1, characterised in that the oral antidiabetically active substances used are

a) 4-[2-(aroylamino)ethyl]-benzoic acids of general formula (I)

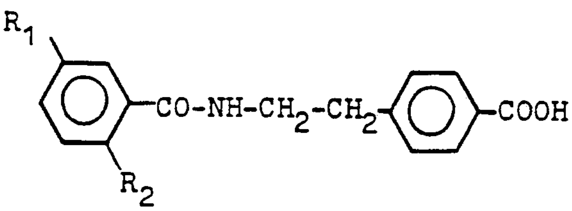

(I)

wherein $R_1$ represents a halogen atom, preferably a chlorine atom and $R_2$ represents an alkoxy group with 1 to 3 carbon atoms, or a piperidin-1-yl or the octamethyleneimino group, or

b) substituted 4-(aralkylaminocarbonylmethyl)-benzoic acids of general formula (II)

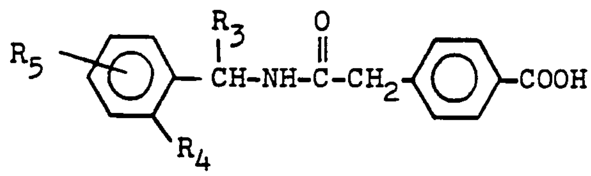

(II)

wherein $R_3$ represents an alkyl group with 1 to 4 carbon atoms, preferably the n-propyl group, or a phenyl group, $R_4$ represents a piperidin-1-yl, pyrrolidin-1-yl or hexamethyleneimino group and $R_5$ represents a hydrogen or halogen atom or a methyl or methoxy group, or mixtures of these substances.

4. Process for the production of oral antidiabetically active preparation forms as claimed in claim 1 containing gliquidone and/or 4-[2-(5-chloro-2-octamethyleneiminobenzoylamino)ethyl]benzoic acid and/or 4-[N-(α-phenyl-2-piperidino-benzyl)-aminocarbonyl-methyl]-benzoic acid and/or 4-[[2-(2-piperidino-phenyl)-1-butyl]-aminocarbonylmethyl]-benzoic acid and a soluble carrier, characterised in that, with the addition of 1 or more basic excipients, the active substance or substances is or are dissolved in a solvent, whilst the molar ratio of active substance to basic excipient must be less than 1:1, polyvinylpyrrolidone and optionally other solubilising substances are dissolved therein and this solution is evaporated to dryness and optionally further processed with other excipients to form the desired preparations.

5. Process as claimed in claims 1 to 4, characterised in that a molar ratio of active substance or mixture of active substances to basic excipient of from 1:1.1 to 1:10 is maintained.

6. Process as claimed in claims 1 to 5, characterised in that the active substance or a mixture of the active substance and the total amount of solubilising substances are used in a ratio of from 1:1 to 1:10 parts by weight.

7. Process as claimed in claims 1 to 6, characterised in that the other solubilising substances used are polyoxyethylene-polyoxypropylene polymers, polyethylene glycols, polyethoxylated sorbitan monooleate, sorbitol, glycerol polyethylene glycoloxy stearates, polyoxyethylene fatty alcohol ethers or mixtures of these substances, whilst the basic excipients used are sodium hydroxide solution, potassium hydroxide solution, tert. sodium phosphate, ammonia, diethanolamine, L-lysine, ethylenediamine or N-methylglucamine.

8. Preparation forms which contain oral antidiabetically active substances, characterised in that acidically or amphoterically reacting active substances together with basically reacting excipients, wherein the molar ratio of active substance to basic excipient must be less than 1:1, are bound to polyvinylpyrrolidone, possibly also in the presence of other solubilising, emulsifying or other substances, and the dried material, possibly together with other excipients, is processed to form pharmaceutical preparations.

9. Antidiabetically active preparation forms as claimed in claim 8, containing as further solubilising and/or emulsifying substances polyoxyethylene-polyoxypropylene polymers, polyethylene glycols, polyethoxylated sorbitan mono-oleates, sorbitol, polyoxyethylene fatty alcohol ethers and glycerol polyethylene glycoloxy stearates or mixtures of these substances, and as basic excipients sodium hydroxide solution, diethanolamine, ethylenediamine, L-lysine or N-methylglucamine, in a ratio of active substance to basic excipient of from 1:1.1 to 1:10 parts by weight.

**Claims for the Contracting State: AT**

1. Process for producing oral preparation forms containing antidiabetically active substances, characterised in that acidically or amphoterically reacting active substances are dissolved with the aid of basic excipients in the presence of polyvinylpyrrolidone and optionally other solubilising substances in a solvent, whilst the molar ratio of active substance to basic excipient must be less than 1:1, the solution is

evaporated to dryness and optionally further processed with other excipients to produce the desired preparation forms.

2. Process as claimed in claim 1, characterised in that oral antidiabetically active sulphonylureas are used.

3. Process as claimed in claim 1, characterised in that the oral antidiabetically active substances used are

a) 4-[2-(aroylamino)ethyl]-benzoic acids of general formula (I)

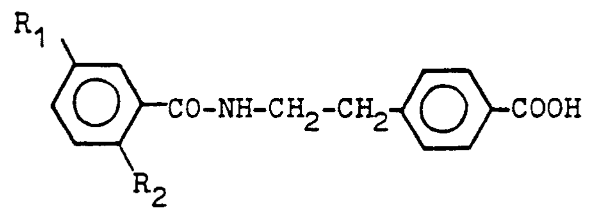

(I)

wherein $R_1$ represents a halogen atom, preferably a chlorine atom and $R_2$ represents an alkoxy group with 1 to 3 carbon atoms, or a piperidin-1-yl or the octamethyleneimino group, or

b) substituted 4-(aralkylaminocarbonylmethyl)-benzoic acids of general formula (II)

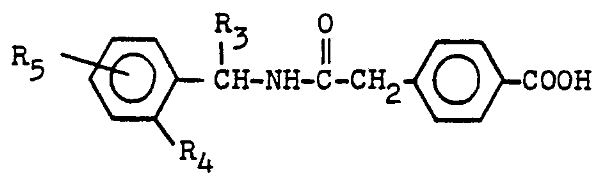

(II)

wherein $R_3$ represents an alkyl group with 1 to 4 carbon atoms, preferably the n-propyl group, or a phenyl group, $R_4$ represents a piperidin-1-yl, pyrrolidin-1-yl or hexamethyleneimino group and $R_5$ represents a hydrogen or halogen atom or a methyl or methoxy group, or mixtures of these substances.

4. Process for the production of oral antidiabetically active preparation forms as claimed in claim 1 containing gliquidone and/or 4-[2-(5-chloro-2-octamethyleneiminobenzoylamino)ethyl]benzoic acid and/or 4-[N-(α-phenyl-2-piperidino-benzyl)-aminocarbonyl-methyl]-benzoic acid and/or 4-[[2-(2-piperidino-phenyl)-1-butyl]-aminocarbonylmethyl]-benzoic acid and a soluble carrier, characterised in that, with the addition of 1 or more basic excipients, the active substance or substances is or are dissolved in a solvent, whilst the molar ratio of active substance to basic excipient must be less than 1:1, polyvinylpyrrolidone and optionally other solubilising substances are dissolved therein and this solution is evaporated to dryness and optionally further processed with other excipients to form the desired preparations.

5. Process as claimed in claims 1 to 4, characterised in that a molar ratio of active substance or mixture of active substances to basic excipient of from 1:1.1 to 1:10 is maintained.

6. Process as claimed in claims 1 to 5, characterised in that the active substance or a mixture of the active substances and the total amount of solubilising substances are used in a ratio of from 1:1 to 1:10 parts by weight.

7. Process as claimed in claims 1 to 6, characterised in that the other solubilising substances used are polyoxyethylene-polyoxypropylene polymers, polyethylene glycols, polyethoxylated sorbitan monooleate, sorbitol, glycerol polyethylene glycoloxy stearates, polyoxyethylene fatty alcohol ethers or mixtures of these substances, whilst the basic excipients used are sodium hydroxide solution, potassium hydroxide solution, tert. sodium phosphate, ammonia, diethanolamine, L-lysine, ethylenediamine or N-methyl-glucamine.

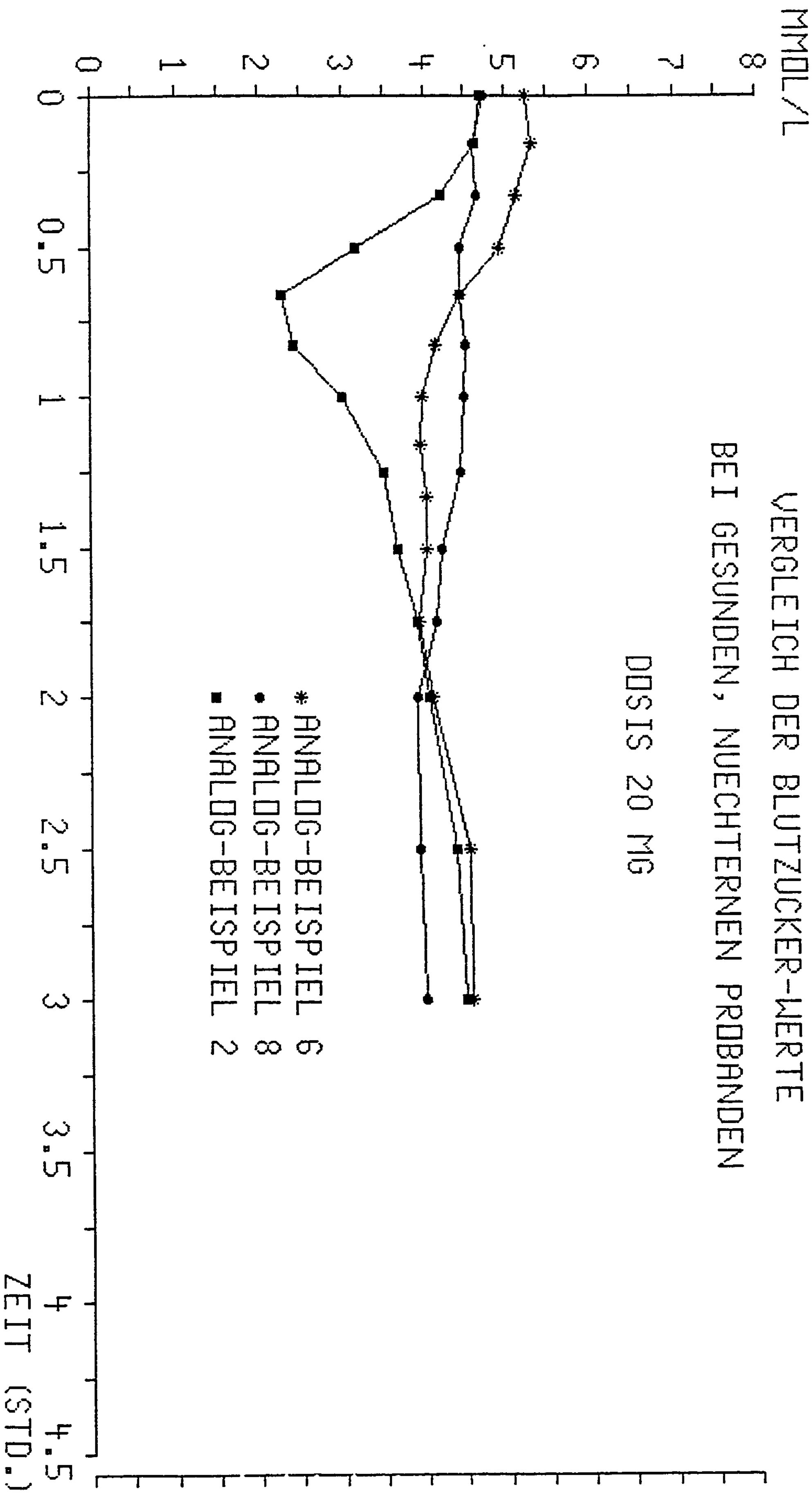
ABB. 1
VERGLEICH DER BLUTZUCKER-WERTE
BEI GESUNDEN, NUECHTERNEN PROBANDEN
DOSIS 20 MG
MMOL/L
0
1
2
3
4
5
6
7
8
0
0.5
1
1.5
2
2.5
3
3.5
4
4.5
ZEIT (STD.)
* ANALOG-BEISPIEL 6
• ANALOG-BEISPIEL 8
▪ ANALOG-BEISPIEL 2